# EUROPÄISCHE PATENTANMELDUNG

(11) **EP 1 429 124 A1**
(43) Veröffentlichungstag der Anmeldung: **16.06.2004**
(21) Anmeldenummer: 03022202.0
(22) Anmeldetag: 30.09.2003
(51) Int. Cl.: G01F 23/26, G01N 33/28

(54) **Verfahren und Schaltungsanordnung zum kapazitiven Erfassen und/oder Bestimmen der Beschaffenheit eines Mediums**

(30) Priorität: 13.12.2002 DE 10258417
(71) Anmelder: Beru AG, 71636 Ludwigsburg (DE)
(72) Erfinder: Bantzhaff, Ralf, 74343 Sachsenheim (DE); Marto, Arno, 71263 Weil der Stadt (DE); Krahl, Jürgen, 96450 Coburg (DE); Munack, Axel, 38531 Rötesbüttel (DE)
(74) Vertreter: Pohlmann, Eckart, Dipl.-Phys.

(57) **Zusammenfassung**

Verfahren und Schaltungsanordnung zum Betreiben eines kapazitiven Mediensensors zur Erfassung und Unterscheidung von Medien. Der kapazitive Mediensensor umfasst einen Kondensator, insbesondere einen Zylinderkondensator, in dessen Spalt ein Medium eingeführt werden kann. Der Kondensator wird mit einer niederfrequenten Taktfolge, die von einem Taktgenerator (6) vorgegeben wird, mit einer Gleichspannung (U_{V}) aufgeladen und der zeitliche Anstieg der Ladespannung des Kondensators wird über einen monostabilen Multivibrator (7) in Impulse umgewandelt, der einen Eingang (E_{T}) für die Taktsignale des Taktgenerators (6), einen Eingang (E_{S}), an dem die Spannung des Kondensators liegt, und einen Ausgang A aufweist, der mit dem Eingang eines Tiefpassfilters (8) verbunden ist, dass die Ausgangsimpulse des monostabilen Multivibrators (7), deren Dauer zur Kapazität des Kondensators proportional sind, in eine Signalspannung umwandelt, die sich linear mit der Kapazität des Kondensators ändert.

## Beschreibung

Die Erfindung betrifft ein Verfahren zum Erfassen und/oder Bestimmen der Beschaffenheit eines Mediums mit einenem kapazitiven Mediensensor, der einen Kondensator umfasst, in dessen Spalt das Medium eingeführt wird, sowie eine Schaltungsanordnung zur Durchführung dieses Verfahrens.

Mediensensoren werden auf dem Gebiet der Kraftfahrzeugtechnik, beispielsweise zur Kraftstofferkennung, zur Ermittlung einer Medienqualtität insbesondere der Ölqualität und zur Erkennung und Erfassung des Auftretens von Wasser, benutzt.

Aus der DE 100 60 419 C2 ist ein Mediensensor, insbesondere ein Wassersensor bekannt, der in Form eines elektrischen Widerstandes ausgebildet ist, dessen Ohmscher Widerstand von dem zu erfassenden Medium abhängt. Ein derartiger Medien- oder Wassersensor kann Medien über die verschiedenen Leitfähigkeiten unterscheiden und dient insbesondere zum Schutz des Einspritzsystems eines Kraftfahrzeuges vor dem Eindringen von Wasser.

Derartige Sensoren sind jedoch nur in der Lage, verschiedene Medien zu unterscheiden, eine Aussage über Art oder Qualität eines Mediums können sie jedoch kaum liefern.

Es sind auch kapazitive Mediensensoren bekannt, die beispielsweise zur Bestimmung der Ölqualität oder der Kraftstoffart dienen und die die Elektrizitätskonstante eines Mediums erfassen. Sie erlauben damit eine genauere Klassifizierung.

Kapazitive Mediensensoren arbeiten jedoch mit hohen Frequenzen und oft mit Brückenschaltungen, die bezüglich der elektromagnetischen Verträglichkeit sehr störanfällig sind und zudem als Sender wirken können.

Die der Erfindung zu Grunde liegende Aufgabe besteht demgegenüber darin, ein Verfahren der eingangs genannten Art sowie eine Schaltungsanordnung zur Durchführung dieses Verfahrens anzugeben, mit denen mittels des selben Sensors sowohl das Auftreten eines bestimmten Mediums als auch die Bestimmung der Beschaffenheit, insbesondere der Art und/oder Qualität eines Mediums möglich ist.

Diese Aufgabe wird gemäß der Erfindung durch ein Verfahren, das im Patentanspruch 1 angegeben ist, und durch eine Schaltungsanordnung gelöst, die im Patentanspruch 2 angegeben ist.

Mit dem erfindungsgemäßen Verfahren und der erfindungsgemäßen Schaltungsanordnung ist es zum einen möglich, die Beschaffenheit eines bestimmten Mediums, beispielsweise eines verwendeten Kraftstoffes zu bestimmen und zu kennen, um die künftigen Abgasvorschriften einhalten zu können, was insbesondere für mit Dieselkraftstoff oder Biodieselkraftstoff betriebene Fahrzeuge gilt, und kann darüber hinaus das Einspritzsystem vor einer Korrosion geschützt werden, da eine Warnung bewirkt werden kann, wenn im Kraftstofffilter ein bestimmter Wasserstand erreicht ist. Beide Funktionen sind bei dem erfindungsgemäßen Verfahren und der erfindungsgemäßen Schaltungsanordnung mit ein und demselben Sensor erfüllbar.

Im Folgenden wird anhand der zugehörigen Zeichnungen ein besonders bevorzugtes Ausführungsbeispiel der Erfindung näher beschrieben. Es zeigen
Fig. 1 in einer Schnittansicht einen kapazitiven Mediensensor,
Fig. 2 in einem Blockschaltbild den Aufbau eines Ausführungsbeispiels der erfindungsgemäßen Schaltungsanordnung und
Fig. 3 in einem Zeitdiagramm die in der Schaltungsanordnung von Fig. 2 auftretenden Signale.

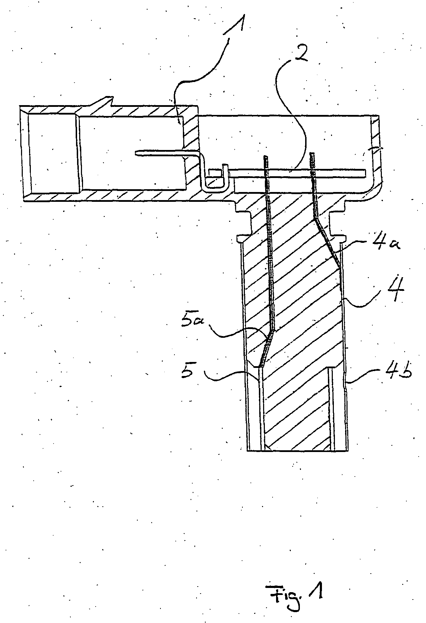

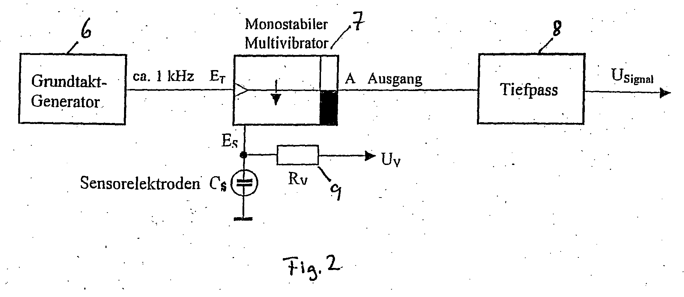

In Fig. 1 ist ein Mediensensor dargestellt, der zur kombinierten Erkennung eines Kraftstoffart sowie des Wasserstandes auf dem Gebiet der Kraftfahrzeugtechnik verwandt wird. Der in Fig. 1 dargestellte Mediensensor umfasst einen Kondensator, vorzugsweise aus koaxialen Hohlkörperelektroden, insbesondere einen Zylinderkondensator aus einer Messelektrode 5 und einer Abschirmelektrode 4, dessen Kapazität unter anderem durch die Oberflächen der Messelektrode 5 und den Spalt zwischen den Elektroden 4 und 5 bestimmt ist. Beide Elektroden 4 und 5 sind über Anschlussdrähte 4a und 5a mit einer Auswerteschaltung 2 verbunden, die im Einzelnen in Fig. 2 dargestellt ist.

Zwischen den Elektroden 4 und 5 ist ein Spülloch 4b vorgesehen, durch das ein Medium in den Elektrodenspalt eintreten kann, so dass es über seine Dielektrizitätskonstante ε gleichfalls die Kapazität des Kondensators bestimmt. Die oben beschriebenen Bauteile sind in einem Gehäuse 1 untergebracht.

Durch die Lage der Messelektrode 5 und des Spülloches 4b ist die Position bestimmt, an der bei der Bestimmung des Wasserstandes eine Warnung erfolgt. Dazu wird der Sensor stehend mit nach oben gerichteten Elektroden 4 und 5 in einem Raum montiert, der von Kraftstoff durchströmt wird und in dem sich andererseits Wasser ansammeln kann. Das ist beispielsweise im Sumpf eines Kraftstofffilters der Fall.

Die Auswerteschaltung 2, die auf einer Leiterplatte angeordnet sein kann, hat einen Aufbau, wie er beispielsweise in Fig. 2 dargestellt ist.

Um den Sensor zur Unterscheidung beispielsweise von fossilem Dieselkraftstoff DK, Biodieselkraftstoff wie beispielsweise Rapsölmethylester RME aber auch anderen Carbonsäurealkylestern wie Sojaölmethylester, Palmölmethylester und ähnlichen Kraftstoffe sowie Wasser verwenden zu können, wird auf die verschiedenen dielektrischen Eigenschaften dieser Medien zurückgegriffen. Die relative Dielektrizitätskonstante ε für Biodiesel liegt bei ca. 4, bei mineralischem Diesel bei ca. 2 und bei Wasser bei ca. 80.

Bei konstanter Geometrie eines mit einer Messflüssigkeit gefüllten Kondensators ändert sich seine Kapazität in gleichem Verhältnis wie die Dielektrizitätskonstante ε der Flüssigkeit, so dass sich aus einer Kapazitätsmessung auf die Dielektrizitätskonstante ε der Flüssigkeit und damit auf die Art der Flüssigkeit schließen lässt.

Auf Grund der oben beschriebenen Bauform des Mediensensors sowie der zur guten Durchströmung des Sensors auch bei hoher Viskosität eines Mediums, wie z. B. bei tiefen Temperaturen des Kraftstoffs, erforderlichen Flüssigkeitsmindestquerschnitte ergibt sich eine Kapazität des Kondensators des Sensors von C_{S} = ε · 2 pF. Die nachgeschaltete Messschaltung muss daher in der Lage sein, Kapazitäten von 4 bis 9 pF linear zu messen. Diese Linearität ist erforderlich, damit auch Gemische von RME und DK erfasst werden können. Eine Kapazität von über 160 pF, wie beispielsweise bei Wasser, muss die Schaltungsanordnung ebenfalls sicher verarbeiten können und durch einen außerhalb des linearen Bereichs liegenden Ausgangssignalwert anzeigen können.

Der Kondensator des oben beschriebenen Sensors ist in Form eines Zylinderkondensators ausgeführt, dessen Abschirmelektrode 4 an Massepotenzial liegt. Die Messelektrode 5 hat maximal Versorgungsspannung. Da die Messung kapazitiv erfolgt, sind isolierende Oberflächenbeschichtungen auf den Elektroden 4, 5 möglich. Ein elektrisch leitender Kontakt zwischen den Elektroden 4, 5 und dem Medium kann auf diese Weise vermieden werden, wenn das erforderlich ist.

Der Grundaufbau der Schaltungsanordnung zum Betreiben des in Fig. 1 dargestellten Mediensensors, der in Fig. 2 dargestellt ist, umfasst einen monostabilen Multivibrator 7 mit einem Sensorelektrodeneingang E_{S}, der mit der Messelektrode 5 des in Fig. 1 dargestellten Mediensensors verbunden ist. Der monostabile Multivibrator 7 wird von einem Grundtaktgenerator 6 getaktet, der mit einer Niederfrequenzentaktung von beispielsweise 1 kHz arbeitet. Das Ausgangssignal des Grundtaktgenerators 6 liegt am Eingang E_{T} des monostabilen Multivibrators 7. Der Ausgang A des monostabilen Multivibrators 7 ist mit einem Tiefpassfilter 8 verbunden, das ein Ausgangssignal U_{SIGNAL} liefert.

Die Abschirmelektrode 4 des Mediensensors liegt an Masse. Wie es weiter in Fig. 2 dargestellt ist, liegt die Messelektrode 5 des Mediensensors über einen Vorwiderstand R_{V} 9 an einer Versorgungsspannung U_{V}.

Die in Fig. 2 dargestellte Schaltungsanordnung arbeitet in der folgenden Weise:

Im Ruhezustand liegt der Eingang E_{S} auf 0 V und somit Massepotenzial, so dass die Messelektrode 5 über den Eingang E_{S} niederohmig auf Massepotenzial liegt. Beide Elektroden 4, 5 sind in diesem Zustand spannungsfrei.

Zu Beginn jedes Grundtaktes, der vom Generator 6 kommt, wird der Eingang E_{S} auf hochohmig geschaltet, und wird damit ein Ladevorgang des Kondensators C_{S} des Mediensensors über den Vorwiderstand R_{V} begonnen, der an die Versorgungsspannung U_{V} angeschlossen ist. Gleichzeitig kommt der Ausgang A des monostabilen Multivibrators 7 auf das_Versorgungsspannungspotenzial. Der Ladevorgang des Kondensators C_{S} endet, wenn die Spannung an der Messelektrode 5 und damit am Signaleingang E_{S} beispielsweise einen Wert von 63,2 % der Versorgungsspannung U_{V} erreicht. Diese Spannung wird im Folgenden als Schwellenspannung bezeichnet. Gleichzeitig mit dem Ende des Ladevorgangs geht der Ausgang A wieder auf Massepotenzial zurück und wird die Messelektrode 5 über den Eingang E_{S} wieder niederohmig auf Massepotenzial gelegt. Dadurch wird der Kondensator C_{S} entladen.

Die sich durch diesen Arbeitsvorgang ergebende Länge tₛ des Impulses am Ausgang A des Multivibrators steht in dem folgenden linearen Zusammenhang zur Sensorkapazität: tₛ = R_{V} · C_{S}. Zwischen der Sensorkapazität und der Impulsdauer besteht daher ein linearer Zusammenhang.

Dieser lineare Zusammenhang ist auch bei anderen Schwellenspannungen als 0,632 · U_{V} gegeben, wobei in diesem Fall der Proportionalitätsfaktor nicht mehr gleich 1 ist.

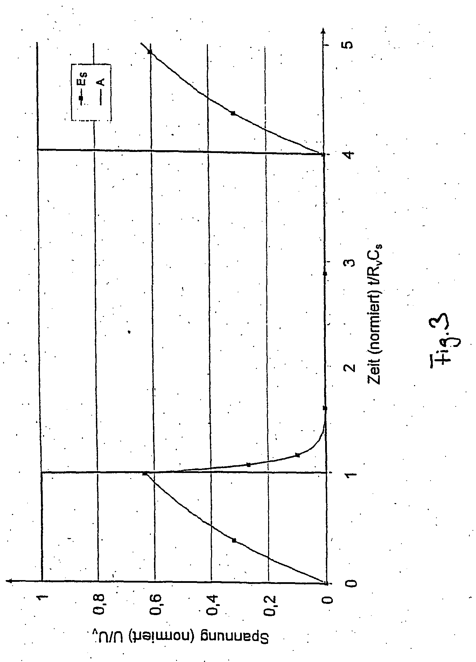

Fig. 3 zeigt in einem Zeitdiagramm den zeitlichen Verlauf der Signalspannungen an den Anschlüssen E_{S} und A. Bei einem Grundtakt von ca. 1 kHz beträgt der in Fig. 3 dargestellte Zeitausschnitt, d. h. die normierte Zeit von 0 bis 5, folglich ca. 1,25 ms.

Das Ausgangssignal am Ausgang A des Multivibrators 7 liegt am Tiefpassfilter 8, das durch Tiefpassfilterung aus dem gepulsten Ausgangssignal des Multivibrators 7 eine linear mit der Kapazität C_{S} des Kondensators des Mediensensors veränderliche Signalspannung U_{SIGNAL} bildet, die dann nur noch in den gewünschten Spannungsbereich durch Verstärkung oder Offset-Korrektur umzuwandeln ist.

Dieses Ausgangssignal hat einen analogen Bereich für den Anteil von Biodiesel, der beispielsweise von 0 bis 100 % bei 0,4 bis 4,5 V liegt und zudem einen Signalbereich, der als Wasserwarnung zu interpretieren ist.

Die Erfassung von Wasser mittels der in Fig. 2 dargestellten Schaltungsanordnung kann in 2-facher Weise erfolgen.

Wasser hat eine sehr hohe Dielektrizitätskonstante. Dadurch wird im Fall nicht elektrisch leitender Elektrodenoberflächen des Sensors der vom monostabilen Multivibrator 7 erzeugte Impuls länger als die eingestellte Dauer des Grundtaktes vom Grundtaktgenerator 6, so dass der Ausgang A dauerhaft auf einem hohen Pegel gehalten wird.

Bei leitfähigen Elektroden bewirkt die elektrische Leitfähigkeit des Wassers einen Kurzschluss der Elektroden. In diesem Fall wird die Schwellenspannung nie erreicht, so dass der Ausgang A ebenfalls dauerhaft auf einem hohen Pegel bleibt.

Die Tatsache, dass der Ausgang A dauerhaft auf einem hohen Pegel bleibt, kann als Indiz für die Erfassung von Wasser herangezogen werden.

Da RME hygroskopisch ist und somit eine geringe Menge an Wasser ohne Phasentrennung einlagern kann, führt dieses Wasser nicht zu einer Veränderung der Anzeige des Mediensensors.

Mit dem oben beschriebenen Verfahren und der oben beschriebenen Anordnung können somit verschiedene Medien, wie beispielsweise Dieselkraftstoff, Biodieselkraftstoff und Wasser auf Grund ihrer verschiedenen Dielektrizitätskonstanten unterschieden werden. Biodieselkraftstoff und Dieselkraftstoff sind darüber hinaus in beliebigem Verhältnis mischbar, wobei die Dielektrizitätskonstante des Gemisches einen linearen Verlauf über das Mischungsverhältnis zeigt.

Zur Durchführung des erfindungsgemäßen Verfahrens wird eine Schaltungsanordnung vorgeschlagen, die lediglich drei Bauelemente, nämlich einen Taktgenerator 6, beispielsweise in Form einer CMOS-IC für Uhren mit Quarzoszillator, einen monostabilen Multivibrator 7 ebenfalls in Form einer CMOS-IC und ein Tiefpassfilter 8 in Form einer RC-Kombination umfasst, die die Ausgangsspannung des Kondensators des Mediensensors verarbeiten, der einen Elektrodenaufbau mit zylindrischer Bauform hat.

Auf Grund der niederfrequenten Signale und der Abschirmung durch die äußere Abschirmelektrode 4 des Mediensensors, die auf Massepotenzial liegt, zeigt die Anordnung eine geringe Empfindlichkeit hinsichtlich der elektromagnetischen Verträglichkeit.

Der Aufbau der Elektroden und der nachgeschalteten Mikroelektronischen Komponenten ist einfach.

Es wird lediglich ein einziges und zwar analoges Ausgangssignal gebildet, das proportional zum Mischungsverhältnis der verschiedenen Medien, beispielsweise der verschiedenen Kraftstoffarten ist.

Mit dem erfindungsgemäßen Verfahren und der erfindungsgemäßen Schaltungsanordnung kann in Verbindung mit dem Mediensensor auch eine Wasserwarnung erzielt werden, wobei für beide Funktionen der Unterscheidung von Medien und der Warnung nur ein Eingang an der Auswerteschaltungsanordnung, nämlich am Multivibrator 7 benötigt wird.

Da der Mediensensor ein kapazitiver Sensor ist, können die Elektroden isoliert sein, so dass keine elektrochemischen Reaktionen im Medium, beispielsweise im Kraftstoff ablaufen.

Grundsätzlich liegt darüber hinaus ein lineares Verhalten der Signalausgangsspannung in Abhängigkeit von Dielektrizitätskonstanten des Mediums zwischen den Sensorelektroden vor. Die Taktfrequenz und die Ausgangsspannungstransformation der Ausgangsspannung U_{SIGNAL} können jedoch so gewählt werden, dass dieses lineare Verhalten nur für den Bereich der Reinkraftstoffe RME und DK zutrifft, während das Vorhandensein von Wasser zu einem Ausgangssignal mit Maximalpegel führt.

## Patentansprüche

1. Verfahren zum Erfassen und/oder Bestimmen der Beschaffenheit eines Mediums mit einem kapazitiven Mediensensor, der einen Kondensator umfasst, in dessen Spalt ein Medium eingeführt wird, **dadurch gekennzeichnet, dass**
der Kondensator mit einer niederfrequenten Taktrate mit einer Gleichspannung aufgeladen wird,
der zeitliche Anstieg der Ladespannung des Kondensators in Impulse umgewandelt wird, deren Dauer zur Kapazität des Kondensators proportional ist, und
die Impulse in eine sich mit der Kapazität linear ändernde Signalspannung umgewandelt werden.

2. Schaltungsanordnung zur Durchführung des Verfahrens nach Anspruch 1, **gekennzeichnet durch** einen Taktgenerator (6), dessen Taktausgangssignal an einem Multivibrator (7) liegt, der einen Signaleingang (E_{S}) aufweist, der mit der Elektrode des Mediensensors verbunden ist, an dem eine Ladespannung liegt, und über den diese Elektrode des Mediensensors hochohmig und niederohmig geschaltet werden kann, und ein Tiefpassfilter (8), an dem das Ausgangssignal des Multivibrators (7) liegt und das an seinem Ausgang (A) eine Signalspannung liefert, die zur Kapazität des Mediensensors proportional ist.
